# EUROPEAN PATENT APPLICATION

(11) **EP 3 372 255 A1**
(43) Date of publication of application: **12.09.2018**
(21) Application number: 18159793.1
(22) Date of filing: 02.03.2018
(51) Int. Cl.: A61L 27/18, A61L 27/54, A61L 29/06, A61L 29/16, A61L 31/02, A61L 31/06, A61L 31/16

(54) **ANTIBACTERIAL SURFACE MODIFICATION FOR MEDICAL DEVICES**

(30) Priority: 09.03.2017 EP 17160207
(71) Applicant: Leibniz-Institut für Plasmaforschung und Technologie e.V., 17489 Greifswald (DE)
(72) Inventor: Polak, Martin, 17489 Hinrichshagen OT Hof II (DE); Hempel, Frank, 17489 Greifswald (DE); Garkas, Wagdi, 17489 Greifswald (DE); Weltmann, Klaus-Dieter, 18609 Binz (DE)
(74) Representative: Schulz Junghans Patentanwälte PartGmbB

(57) **Abstract**

The invention relates to a medical device comprising a biocompatible material having an exposed surface prone to biofilm growth. The biocompatible material comprises a doping of an antibacterial metal compound. A concentration of the doping compound ranges from 1% to 30% (w/w) within an outer layer adjacent to the exposed surface, and is essentially zero within a bulk layer of the biocompatible material.

## Description

Urinary tract catheters, stents or other medical devices for conducting urine often suffer from encrustation, i.e., adhesion of crystalloids or colloids to a surface, caused by the crystallization of struvite and apatite in the presence of urease-producing microorganisms. The formation of biofilms on the surface of the devices, and therefore the generation of a local change in pH to an alkaline environment in the urinary tract, favor the formation of encrustations. While the suppression of biofilms is always desirable, it is necessary to prevent pH-changes to avoid the formation of encrustations.

Methods for obtaining surfaces impeding microbial growth are known in the art. These include the inclusion of an anti-microbial additive in polymers, metals and other surface materials, or including these additives in paints and surface coatings, or physical or chemical vapor deposition (PVD/CVD).

Silver or copper particles may be added to the melt of metals or polymers during manufacture. The disadvantage of such processes is that only low surface concentrations can be achieved, and most of the anti-microbial compound is lost in the bulk of the material. Furthermore, additives can impact negatively on the hardness, resistance to abrasion and corrosion of the bulk material. Therefore, the use of this process is restricted to niche applications.

Sol-gel processes are known for applying anti-microbial agents bound in a gel matrix to a surface. This approach works well for applying large quantities, however, the agent needs to be fixed by thermal processes for achieving abrasion resistance. Thermally labile materials do not allow thermal treatment.

WO208/027720 A1 shows anti-microbial beryllium alloys with copper, nickel and cobalt and its use in a fluid drainage device.

US 5837275 A discloses a medical device comprising an anti-bacterial coating that is obtained by magnetron sputtering. Here, instead of depositing the anti-microbial compound throughout the bulk material of the medical device, only a coating on an outer surface of the medical device is disclosed. US 5837275 particularly deals with the specific way of generating a disorder in the coating with specific grain sizes. However, while the release of an anti-microbial agent basically relies on the grain size, the coating can be damaged, impairing this effect.

WO 2007/087269 A2 teaches a cathodic arc ion plasma deposition method, for preparing modified metal coatings for forming anti-microbial surfaces on medical devices. Furtermore, WO 2007/087269 A2 teaches how to treat a surface of a medical device such that a better adhesion of a metal coating on the medical device is achieved by imbedding some of the metal ions in the medical device, wherein eventually a complete surface coating of the device is produced. Such a surface coating has a very high concentration of the deposited metal, which renders the device also vulnerable to scratches, which reduces the anti-microbial effect of the coating.

US 2010/0264016 A1 teaches a high power impulse magnetron sputtering method (HiPIMS) that does not require the presence of an inert gas in order to initiate or to sustain a plasma formation.

However, the HiPIMS method alone is not suited for producing medical devices that are doped with an anti-microbial compound for continuous and sustained release.

The objective of the present invention is to provide a surface of a medical device that resists the formation of encrustation when exposed to fluids inside the human body. The surface of the medical device of the invention avoids the disadvantages of the devices known in the art. Another objective of the present invention is the provision of a process for making such a surface. These objectives are attained by the subject-matter of the independent claims.

### Terms and definitions

The term silicone in the context of the present specification refers to a siloxane (Si-O-Si-O-Si...) containing polymer.

The term medical silicone in the context of the present specification refers to a silicone used in and having regulatory approval for medical use.

The term polyurethane in the context of the present specification refers to a polymer made of monomers joined by carbamate linkages.

The term polyvinylchloride in the context of the present specification is used as is common in the art; it refers to the polymer catalogued under CAS No. 9002-86-2.

The term shape memory alloy in the context of the present specification refers to a metallic material exhibiting, qualitatively, the shape memory effect known from NiTiNol and NiTiCo.

The term NiTiNol in the context of the present specification refers to a shape memory alloy of titanium and nickel, comprising approximately 50 to 60 weight percent (w/w) nickel, the rest being titanium.

The term NiTiCo in the context of the present specification refers to a shape memory alloy of titanium, cobalt and nickel, comprising approximately 50 % to 60 % (w/w) nickel, the rest being titanium.

Titanium alloy in the context of the present specification refers to typical Titanium alloys used as load bearing implants as specified in ASTM Grade 1-35.

Load bearing material in the context of the present specification refers to materials used for load bearing implants (e.g., hip joints, knee joints, metal plates for plastic surgery). Examples include, but are not limited to, load bearing polymers such as ultra-high-molecular-weight polyethylene, metal alloys commonly used in hip and knee replacements (exemplified by, but not limited to, a titanium alloy or a zirconium-niobium alloy (Zr2Nb5)), and ceramics commonly used in hip and knee replacements.

Resorbable material / Mg alloy (e.g., WE 43) in the context of the present specification refers to a material which is bioresorbable inside the human body (e.g., for stents, bone screws).

The term "High Power Impulse Magnetron Sputtering" (HiPIMS) in the context of the present specification refers to a method for physical vapor deposition of thin films, which is based on magnetron sputter deposition.

In contrast to conventional magnetron sputtering techniques, HiPIMS is a pulsed-mode sputtering method that applies short unipolar high-voltage pulses to the magnetron (target). The voltage pulses are in the order of several hundred Volts to several thousand Volts, such as for example 1 kV.

The duty cycle, that is the fraction of a period during which the voltage is applied divided by the fraction of the period during which no voltage is applied, ranges in the order of 5% to 15%, particularly 10%. The operating frequency is typically in the range of 10 Hz - 1 kHz, resulting in pulse durations in the range of tens to hundreds microseconds (1 µs-1000 µs).

These operating parameters lead to peak target power densities of the order of several kilowatts per square centimetre and current densities in the order of 0.1 A/cm2 to 5 A/cm2, while keeping the average target power density low enough to avoid magnetron overheating and target melting.

HiPIMS can be defined as pulsed magnetron sputtering, where the peak power exceeds the time-averaged power by typically two orders of magnitude.

Due to the high peak power, it is possible to generate a highly ionized plasma with large quantities of ionized sputtered material. This in turn leads to favorable properties with respect to the uniformity of the coating, even on complex substrate geometries.

Particularly, surfaces that extend along low flux directions in conventional sputtering techniques can be coated more uniformly with HiPIMS. Thus, HiPIMS is known to result in smooth and dense coatings of the sputtered material.

HiPIMS is known to produce a high-density plasma capable of producing large quantities of ionized sputtered target material within a short time, as compared to conventional, particularly non-pulsed sputtering techniques.

It is noted that the substrate can be kept at a neutral charge or is biased slightly negative, for example at -50 V with respect to the anodes. The negative bias leads to an acceleration of the sputtered ions towards the substrate during the off-times of the duty cycle in HiPIMS.

The term "Plasma Based Ion Implantation" (PBII) in the context of the present specification refers to an ion implantation method in which a substrate, whose surface is to be doped with a dopant, is immersed in a plasma. The substrate is then doped with ions that are extracted from the plasma by means of short negative high-voltage pulses applied to the substrate. The pressure in the plasma chamber ranges particularly from 0.01 Pa to 1 Pa.

The short pulses in the kV-range cause an acceleration of the positively charged plasma ions (comprising the ionized target material and/or the process gas ions, such as argon ions) towards the substrate. Due to the adopted kinetic energy of the plasma ions these ions will be implanted in the substrate when they hit the substrate. The depth of incorporation of the ions depends for example on the voltage applied to the substrate.

The voltage ranges particularly from 1 kV to 100 kV and the pulse duration is in the range of several tens to hundreds microseconds in order to avoid excessive heating of the substrate. PBII advantageously provides homogenous implantation in arbitrarily-shaped substrates.

### Summary of the invention

The surface's outer layer doping with a metal compound leads to an inhibition of a pH value increase on the catheter surface in the urinal tract, which is otherwise associated with formation of uric acid formation, or encrustation of the surface. In addition, it inactivates bacteria and inhibits the formation of a biofilm on the surface. The surface treatment does not affect the mechanical stability of the surface.

A first aspect of the invention relates to a medical device that comprises or at least partially consists of a material that is in contact with a patient. This biocompatible material has an exposed surface, which may be in danger of being subject to a process of encrustation or biofilm growth, or be in other ways prone to biofilm growth. The biocompatible material of the present invention comprises a doping of an antibacterial metal compound and is further characterized in that a concentration of the metal compound in the biocompatible material reaches a significant value within an outer layer adjacent to said exposed surface and is essentially zero within a bulk, particualrly a bulk layer, of said biocompatible material.

In other words, the antibacterial dopant is not homogeneously distributed within the bulk of the material, as could be achieved by mixing the dopant prior to polymerization or other methods of generating the material, but it is contained within a relatively small surface layer on or adjacent to a surface that is in contact with the human or animal body.

The antibacterial dopant is particularly not forming a coating on the surface.

In certain embodiments, the concentration of the dopant ranges from 1% to 30% (w/w) within an outer layer adjacent to said exposed surface, and is essentially zero within a bulk, particualrly a bulk layer of said biocompatible material.

Directly on the surface of the medical device the concentration of the dopant is particularly within 1% to 5% (w/w). The dopant particularly increases form the surface to higher concentrations within the outer layer to values above said range.

The highest concentration of the dopant is particularly with 5 nm to 20 nm away from the surface of the medical device. This renders the medical devices robust against wear and tear such like scratches. The anti-microbial properties of the medical devices are sustained and particularly form a dopant reservoir below the surface of the medical device.

The outer layer extends particularly from slightly below the surface inwards the medical device to depth of 5000 nm.

In certain embodiments, the concentration of the antibacterial metal dopant compound has a maximum value at said exposed surface and decreases, e.g., linearly or exponentially, until a depth of 10 nm to 1000 nm, where the doping compound is undetectable.

According to another embodiment of the invention, the concentration of the metal dopant compound particularly increases within the medical device, particularly within a depth range of 5 nm to 20 nm, above the dopant concentration on the surface to a peak value and then decreases exponentially or linearly.

In certain embodiments, the concentration of the antibacterial metal dopant compound has a maximum value at said exposed surface and decreases, e.g., linearly or exponentially, until a depth of 100 nm to 500 nm, where the doping compound is undetectable.

In certain particular embodiments, that depth where the doping compound is undetectable is 100 nm to 300 nm.

In certain embodiments, the concentration of the antibacterial metal dopant compound has a maximum value in a depth of 10 to 500 nm and decreases, e.g., linearly or exponentially, to a depth of 10 nm to 1500 nm, particularly to a depth of 100 nm to 300 nm, at which depth the doping compound is undetectable.

In certain embodiments, the concentration of the antibacterial metal dopant compound is undetectable on the surface and has a maximum value in a depth of 10 to 500 nm. The concentration thereafter decreases linearly or exponentially at greater depth until the doping compound is undetectable in a depth beyond 800 nm, 1000 nm or 1500 nm. The advantage of this distribution is in the kinetics: the liberation of the dopant sets in after days or weeks and increases continuously thereafter.

In certain embodiments, the antibacterial metal compound is selected from copper, copper oxide, silver, zinc, zinc oxide, cobalt, cobalt oxide, aluminium, aluminium oxide, titanium, titanium oxide, and mixtures thereof.

In certain embodiments, the exposed surface comprises or is essentially made of
a. Silicone, particularly medical silicone, polyurethane, particularly aliphatic polyurethane, more particularly thermoplastic aliphatic polyurethane, polyvinylchloride, polypropylene, polystyrene, latex, teflon or other tissue compatible polymers used in medical devices, or of
b. a shape memory alloy, particularly NiTiNol or NiTiCo or
c. a load bearing material, particularly selected from titanium or a titanium alloy, stainless steel, and a resorbable material like, magnesium and its alloys and hydroxyapatite.

In certain embodiments, the titanium alloy comprises at least 30 % titanium.

In certain embodiments, a coating, particularly a coating having a thickness ranging from 1 nm to 400 nm, more particularly ranging from 10 nm to 100 nm, of said antibacterial metal compound is comprised or applied to said exposed surface. Such coating enables the deposition of a greater amount of material, leading to longer duration of the intended antibacterial effect as well as an increased anti-microbial efficacy.

In certain embodiments, the antibacterial compound of the coating is the same as the antibacterial compound comprised in said outer layer.

In certain embodiments, the medical device is a urinary tract catheter.

In certain embodiments, the medical device comprises a cavity, tubular (sub-) structure or cavity characterized by a lumen enclosed by a surface, and said exposed surface is the outer surface as well as the opposite luminal (inner) surface.

In certain embodiments, the exposed surface is an outer surface opposite to a luminal (inner) surface.

In these embodiments, only the outer surface of a catheter is treated by the method of the invention, or the outer surface comprises the surface according to the invention. Under certain circumstances, the outer surface can be the primary site of incrustations. Under certain circumstances, the (inner) lumen of the catheter is treated by the method of the invention, or the (inner) lumen comprises the surface according to the invention. Under certain circumstances, the (inner) lumen of the catheter can be the primary site of incrustations. The present invention differentiates itself from state of the art processes in that the outer layer, i.e., the region of the catheter material that is adjacent to the surface, is enriched in metal compound dopants such as metallic silver, copper, zinc, aluminium, cobalt, titanium, or their respective oxides. In addition, a thin layer (ranging from one or a few nanometres to several hundred nanometres) of the metallic dopant may exist but is not prerequisite for the encrustation prevention to take effect. This differentiates the process of the present invention, and the surfaces obtained therefrom, from prior art processes, which either proceed by mixing a metal additive into the bulk material prior to polymerization, or only apply a surface coating on the catheter material. The present invention however achieves a continuous transition from the pure bulk material to an outer layer, adjacent to the surface, wherein the dopant concentration increases from zero dopant in the bulk layer to a surface concentration fitted to avoid encrustation. This outer layer, in which the concentration increases from just above zero to the surface concentration, may be one or a few nanometres to several hundred nanometres, or even a micrometer thick.

In certain embodiments, the surface may be covered by a thin coating of pure dopant.

The problem according to the invention is furthermore solved by a process for producing a biocompatible material having an outer layer comprising a doping of an antibacterial metal compound, said process comprising the steps of:
a. mounting a biocompatible material on an electrode in an enclosed space of a magnetron sputtering system comprising a target material comprising the antibacterial metal compound and a magnetron source for magnetron sputtering;
b. reducing the atmospheric pressure of a process gas, particularly argon, in said enclosed space to below 1 Pa particularly to below 0.1 Pa, 0.01 Pa or even 0.001 Pa;
c. applying a first series of particularly intermittent negative voltage pulses particularly in the range from -10 kV to -100 V with respect to a ground potential, to the magnetron source or to the target material so as to create a pulsed plasma within said enclosed space, whereby said target material is released in an atomized state into said enclosed space, wherein at least a fraction of the target material is or becomes singly or multiply ionized in the plasma;
d. applying a second series of negative voltage pulses particularly in the range of -500 kV to -50 V with respect to a ground potential to said electrode, particularly to the biocompatible material, wherein the negative voltage pulses of the second series are time-shifted with respect to the voltage pulses of the first series by at least the duration a voltage pulse of the first series, wherein the ionized target material is accelerated towards said electrode by said voltage pulses of the second series and implanted in the biocompatible material upon impact on the biocompatible substrate, thereby doping the biocompatible material with an antibacterial metal compound.

The above described process particularly combines high power impulse magnetron sputtering (HiPIMS), as particularly described in step c.) and plasma based ion implantation (PBII), as particularly described in step d.), such that the advantages of step c.) and d.) particularly HiPIMS and PBII are synergetically combined. The order of step c) and step d) can be inverted, that is first step d) is performed and then step c). As these steps are usually performed repeatedly, it can be indistinguishable, which step advances the other.

Particularly the high degree of ionization of the sputtered material leads to the implantation of sputtered material into the layer of the biocompatible material and/or the growth of smooth and dense coatings on the biocompatible material.

The first series of pulses generates a high degree and high flux of sputtered material, wherein the second series of pulses leads to a high-rate, smooth implantation in the biocompatible material.

The interleaved application of the second series of voltage pulses with the first series of voltage pulses therefore advantageously combines these two processes to an improved implantation method for biocompatible materials.

Depending on the amplitude and duration of the voltage pulses of the second series, the implantation depth can be controlled. The depth of implantation also depends on other factors such as charge of the sputtered target material, density of the plasma and/or pressure in the enclosed space.

Simultaneously to the implantation process caused by the second series of pulses, the uncharged, sputtered target material can contribute to the coating of the biocompatible material.

Thus, by combining step c.) and step d.), particularly by combining HiPIMS with PBII, the doping of surface layers of the biocompatible material with the target material is facilitated, wherein the surface layers are able to resist wear and microbial growth.

According to another embodiment of the invention, the target material is copper, copper oxide, silver, zinc, zinc oxide, cobalt, cobalt oxide, aluminum, aluminum oxide or a mixture thereof.

These target materials are well suited for the combined application of HiPIMS and PBII and simultaneously provide antibacterial properties to the biocompatible material.

According to another embodiment of the invention, the creation of ionized target material is facilitated by a pulsed-magnetron sputtering method, such as for example HiPIMS.

According to another embodiment oft h einvetion, the creation of ionized target material is facilitated by a high power impulse magnetron sputtering method (HiPIMS).

According to another embodiment of the invention, the implantation of the ionized target material is facilitated by the PBII method.

According to another embodiment of the invetion, the implantation of the ionized target material is facilitated by a plasma-based ion implantation method (PBII).

Wherever alternatives for single separable features such as, for example, a polymer, a metal or a depth profile, are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.
- Fig. 1: shows a schematic view of a medical device having a surface according to the invention.
- Fig. 2: shows a cross section of catheter wall modified by the method of the invention.
- Fig. 3: shows the XPS intensity of the Cu2p peak for different depths achieved with Ar-sputtering.
- Fig. 4: shows AFM images of surfaces modified with different processes performed according to the first parameter set given above, unless specified otherwise.
- Fig. 5: shows the diffraction pattern of a pure coating;
- Fig. 6: shows the anti-microbial effect of the treated surface;
- Fig. 7: shows a schematic depiction of the plasma based implantation apparatus; and
- Fig. 8: shows various polypropylene samples coated by the process according to the invention;
- Fig. 9: shows three depths profiles for catheters that have been treated for different durations with the method according to the invention;
- Fig. 10: shows the anti-bacterial effect of a device doped with copper ions and manufactured with the method according to the invention after 24h;
- Fig. 11: shows the anti-bacterial effect of a device doped with copper ions and manufactured with the method according to the invention after 48h; and
- Fig. 12: shows the anti-bacterial effect of a device doped with zinc and manufactured with the method according to the invention after 48h.

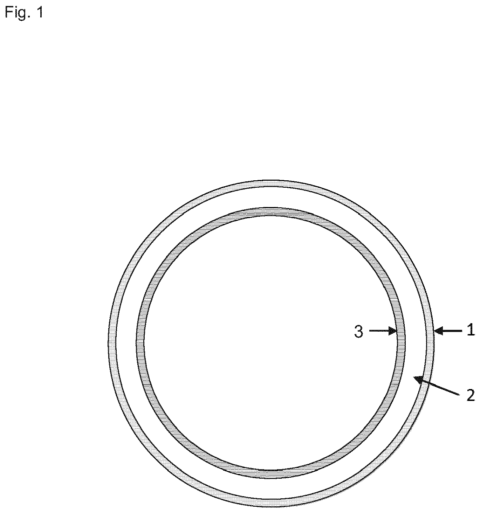

In Fig. 1 a schematic view of a medical catheter having a modified surface according to the invention is shown. The exterior 1 of the catheter comprises a high density of ions implanted in the bulk material of the catheter, wherein away from the exterior surface, unmodified bulk material 2 is located comprising no implanted ions. The interior 3 of the catheter comprises again bulk material comprising a high density of implanted ions.

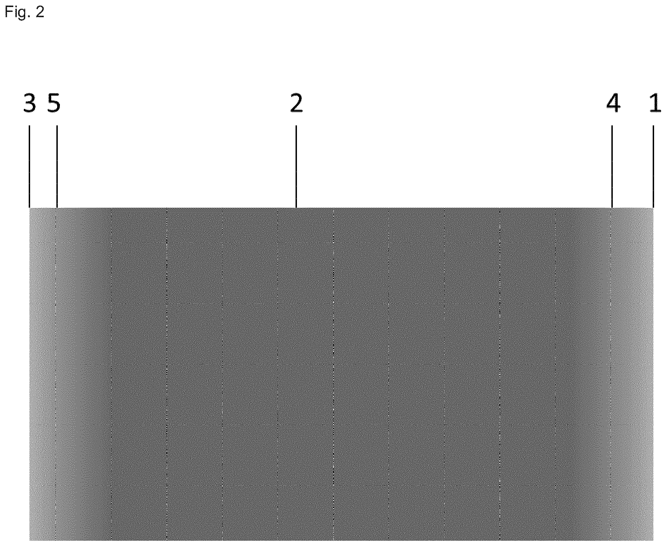

In Fig. 2 a schematic cross-section of the catheter of Fig. 1 is shown. Additionally to the interior 3, the exterior 1 of the catheter as well as the inner core 2 of unmodified bulk material of the catheter, the the plasma-modified zones with implanted ions in the bulk material ranging from the exterior 1 to the depth marked "4" and form the interior 3 to the depth marked "3" are indicated, with respect to the exterior 1 and the interior 3 of the catheter.

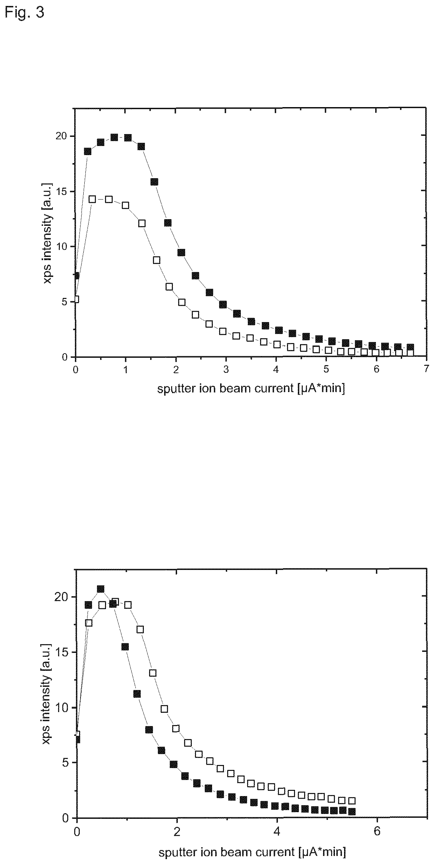

Fig. 3 shows a depth profile of a material treated according to the invention. The sputtering current was measured and integrated over the time resulting in an integrated supttering current having the units of [µA*min] that can be used as a direct depth measurment.

As the integrated sputtering current is related to a depth in the material, the zero on the x-axis corresponds to the surface of the medical device and larger values correspond to an increasing depth of the material. The γ- axis represents the amount of copper deposited. The unit µA * min on the x-axis is used as a direct depth measurement.

The plot with the filled squares refers to the combined method of HiPIMS and PBII, whereas the plot with the hollow squares refers to the coating of the material applying only a HiPIMS method, wherein the HiPIMS pluses, the pulse duration and off-time were identical to the HiPIMS pulses in the combined case.

The pulses for the HiPIMS shown in the diagram in the upper panel (plots with solid and hollow squares) were characterized by a 20 µs positive edge and a 20 µs negative edge and an off-time of 320 µs between the HiPIMS pulses.

The plot comprising the black squares refers to the method where HiPIMS and PBII are combined. The pulses for the BPII shown in the diagram in the upper panel (plot with solid squares) were given by a 20 µs pulse duration, a 20 µs delay time with respect to the HiPIMS pulses and an off-time of 320 µs. This specific parameter set is referred to as the "400 µs" parameter set in the following, as the complete cycle time amounts to 400 µs.

By varying the delay, the voltage and the pulse duration of the HiPIMS and PBII pulses the coating and implantation process can be controlled.

In the lower panel of Fig. 3 the same plots are shown, recorded with the same parameters as the plots in the upper panel but with an off-time of 920 µs between the pulses. Again, the plot comprising the hollow squares refers to the method comprising only HiPIMS pulses, wherein the plot comprising the solid squares refers to the method comprising HiPIMS pulses and PBII pulses. The HiPIMS pulses, pulse duration and off-time were identical for both experiments. This specific parameter set is referred to as the "1000 µs" parameter set in the following, as the complete cycle time amounts to 1000 µs.

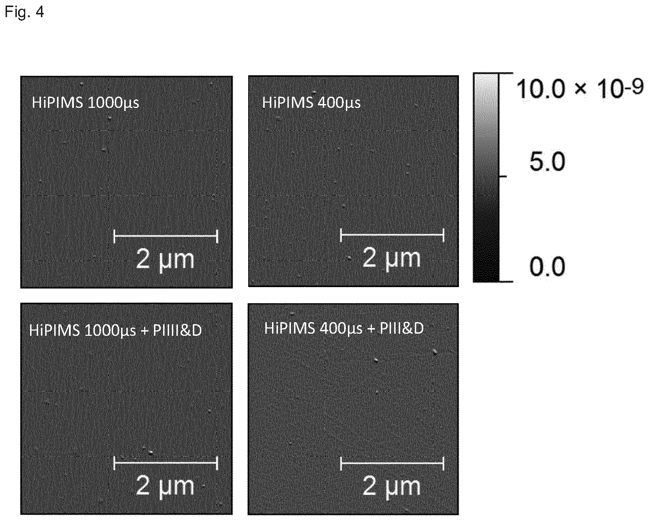

The surface of various samples coated under different conditions is shown in Fig. 4. The surface roughness measured on the samples depicted in Fig. 4 is represented by the following table:

| | HiPIMS 1000 µs | HiPIMS 400 µs |
|---|---|---|
| without PIII&D | 0.46 nm (upper left panel in Fig. 4) | 0.63 nm (upper right panel in Fig. 4) |
| with PIII&D | 0.29 nm (lower left panel in Fig. 4) | 0.22 nm (lower right panel in Fig. 4) |

The scale bar in Fig. 4 refers to a relative elevation in units of meter with respect the lowest point measured in the image.

As can be seen from the table, the method according to the invention (combined HiPIMS and PBII) leads to smoother surfaces in comparison to deposited layers by pure HiPIMS or other physical vapor deposition (PVD) methods as well as galvanic or other wet chemical processes.

The images were obtained by atomic force microscopy (Bruker; tip MPP-11123, area 5µm x 5µm, resolution 512x512 points, frequency 0,5 Hz) in contact mode. The roughness values were determined employing the software Gwyddion.

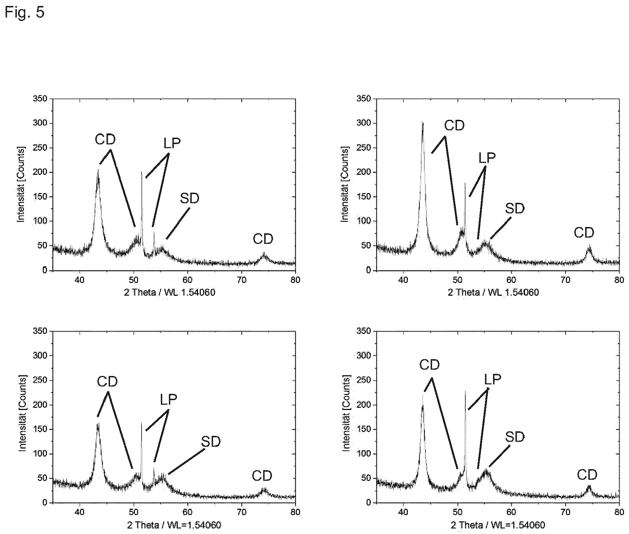

Fig. 5 shows the diffraction pattern of a pure coating performed according to the "400 µs"-parameter set in the upper panel and according to the "1000µs"-parameter set in the lower panel. The plots on the upper and lower right panel show the diffraction patterns achieved with only HiPIMS applied to the sample, wherein the plots on the upper and lower left panel show a surface enhancement using the combination of HiPIMS and PBII. The combination of HiPIMS and PBII generates more copper crystals. The sample generated by the combination of HiPIMS and PBII furthermore exhibits a higher degree of crystallinity and lower polymorphic qualities, which can be seen by comparing the indicated peaks in the diffraction plots. The copper diffraction peak is marked with "CD", the silicon diffraction peak is marked with "SD" and the Laue-peak is marked with "LP" for all plots.

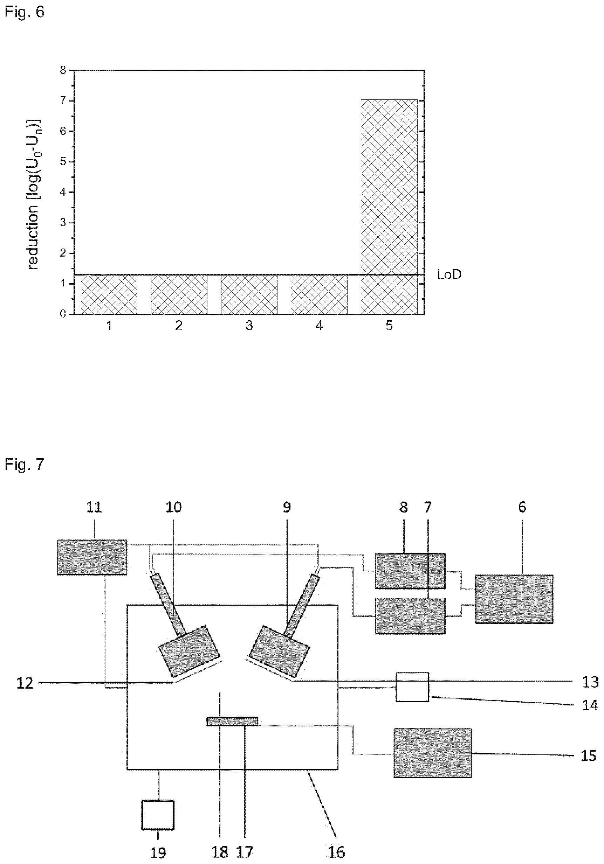

In Fig. 6 the anti-microbial effect of the modified surfaces is shown. The tests were performed with a *S. aureus* suspension in 0.85% NaCl with 1.86E+07 cfu/ml (cfu = colony-forming units). A 50 µl drop was used for the surface contamination and the resulting effect was evaluated after 120 min incubation time by counting the colony-forming units. On the y axis the logarithm of the difference between the number of colony-forming units at time zero U₀ and the number of colony-forming units after 120 min is shown for 4 differently treated surfaces (1 to 4 on the x-axis) and a non-treated surface (5 on the x-axis) is shown.

On the treated surfaces a reduction of six orders of magnitude (99.9999% of cfu) is observed in comparison to the non-treated surface, which corresponds to a reduction below the detection limit (LoD, shown as a horizontal black line).

Specific sample treatment as referred to with the numbers on the x-axis:
Sample 1: "1000ps"-parameter set (HiPIMS);
Sample 2: "1000µs"-parameter set (HiPIMS + PIII&D);
Sample 3: "400µs"-parameter set (HiPIMS);
Sample 4: "400µs"-parameter set (HiPIMS + PIII&D);
Sample 5: untreated ABS control sample;

In Fig. 7 the apparatus for implantation is shown. The apparatus comprises a plasma vacuum chamber 16 that encloses a first magnetron 9, a second magnetron 10 each with a controllable shutter 12, 13.

In the chamber 16 there is also a substrate holder 18 arranged in a plasma zone 18 of the vacuum chamber 16.

The chamber 16 can be evacuated by a vacuum pump unit 19. The pressure inside the chamber is controlled by a pressure control 14. A gas flow control unit 11 regulates the process gas flow into the chamber 16. The substrate holder 17 is connected to a PBII-control unit 15 that is configured for providing the PBII pulses to the electrode, e.g., the substrate holder 17. Each magnetron 9, 10 is connected to its own DC power supply 7, 8, wherein a HiPIMS-control unit 6 controls the first and second magnetron 9, 10 via the DC power supplies 7, 8.

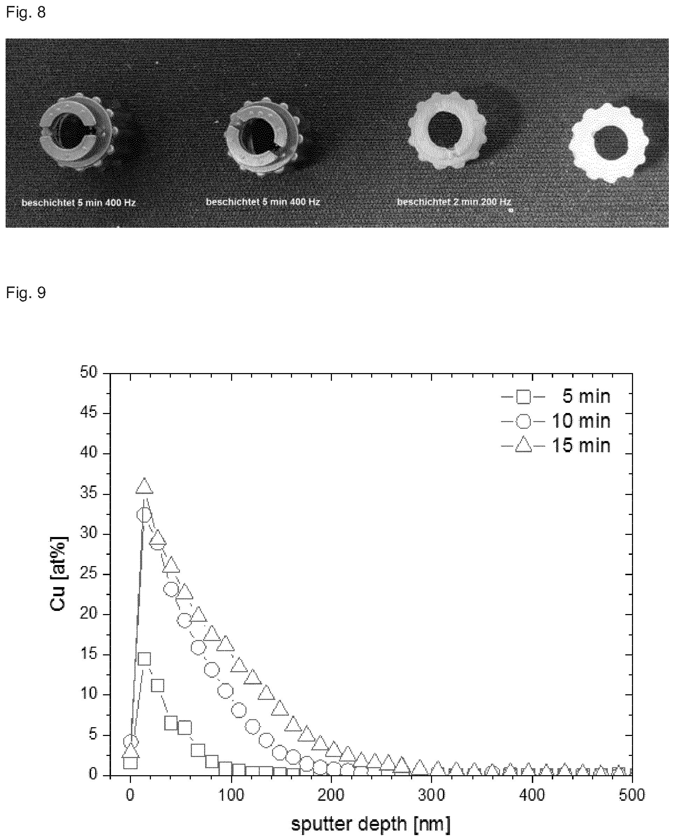

Fig. 8 shows polypropylene samples coated by the process of the invention.

The treatment according to the invention renders this substrate colored. Untreated material appears almost white (rightmost sample shown in Fig. 8). Material treated for 2 min at 200 Hz (second from the right) shows a yellow tinge. The more power was expended during treatment (equivalent to an increased length, or height of the pulses applied, the frequency of repetition of the pulses or the duration of the treatment), the closer the color of the material is to the color of metallic copper. Treatment over 5 min at 400 Hz (leftmost sample) and for 5 min at 400 Hz leads (second from left) to a darker coloring.

### Further examples

### Example 1: Coating and doping of a medical device

In this example, the biocompatible material is a medical device, particularly a catheter. The medical device is placed on a holder, wherein, if the medical device is electrically nonconducting, the electrically conducting electrode that is part of the holder is inserted in the substrate. In case the medical device is electrically conducting, the medical device is connected to a voltage supply for the generation of a series of high-voltage pulses for a PBII method.

Said holder with the medical device is subsequently transferred into the vacuum chamber that forms the enclosed space for the sputtering and implantation process. The vacuum chamber is then evacuated down to a pressure of 0.001 Pa to 1 Pa.

It is possible to provide a process gas such as argon in order to suppress oxidation processes of the medical device that might occur when other, more reactive gases are used.

The vacuum chamber further comprises a DC-magnetron source that is operable in a pulsed-DC mode via a suitable high-voltage pulse generator. Thus, the DC-magnetron source can be used to provide a first series of high-voltage pulses to the magnetron and induce a plasma in the vacuum chamber.

The specific mode of pulsed-DC operation enables the sputtering in the HiPIMS regime that is characterized particularly by high ion densities, by a high ion production rate and by high ion energies, as compared to non-pulsed magnetron sputtering methods.

The coating and doping of the medical device with the target material is facilitated in an interleaved manner.

First, a first negative high-voltage pulse is provided to the magnetron source, inducing a plasma in the vacuum chamber, wherein the plasma comprises argon ions. These argon ions are accelerated towards the negatively charged magnetron and hit the target material that is placed on top of the magnetron source.

The plasma, particularly the argon ions, and later also the atomized target ions, sputter the target material into the plasma, where said atomized target material is ionized by the electrons in the plasma. Here, the magnetic field of the magnetron is particularly advantageous, as it is well-known that by applying a magnetic field to the plasma, the ionizing rate per electron can be increased, as the electrons are confined for a longer time in the relevant region over the magnetron.

Depending on the process parameters, the target material can be multiply ionized.

After said first negative high-voltage pulse has been applied and is decayed, a second negative high-voltage pulse, not necessarily having the same amplitude and duration as the first pulse, is applied to the electrode or directly to the medical device.

This second pulse will cause the ions of the target material in the plasma to accelerate towards the medical device. Upon impact on the medical device, the ionized target material has such a high kinetic energy that the ions are penetrating the surface layers of the medical device. The penetration depth depends again on various process parameters, such as the amplitude of the second voltage pulse, the duration of the second voltage pulse, the mean free path length in the plasma, and the charge of the specific ion.

Once the second voltage pulse has been applied to the medical device or the electrode, again the first high-voltage pulse is applied to the magnetron, in order to replenish the plasma ions for coating and implantation and so on.

Thus, a first series of high-voltage pulses is applied to the magnetron interleaved by a second series of high-voltage pulses applied to the medical device, until a sufficiently high degree of dopant, is incorporated in the surface layers of the medical device.

It is possible to keep the medical device or the electrode on a constant negative bias that is to apply a negative DC-voltage to the medical device and overlay the second series of negative pulses on said negative bias.

The target material is arranged on the magnetron source, wherein the target material might comprise practically any solid substance. The target material can be provided as a powder, or as a mixed target with regions consisting of different compounds.

Also liquids such as medical compounds might be vaporized by the vacuum and incorporated into the surface layer of the medical device with the disclosed process. The same holds true for gases.

As mentioned above, the vacuum chamber might be filled with a process gas such as a noble gas, particularly argon.

The medical device is exposed for several seconds up to several minutes and hours to the disclosed coating and implantation process. The longer the medical device is exposed to the process, the thicker a coating becomes and the concentration of incorporated target material increases accordingly.

Another advantage of the disclosed combination of HiPIMS and PBII is that HiPIMS also exhibits excellent coating properties even for geometries that exhibit regions that are hidden to the so-called line-of-sight. Therefore, the medical device, such as a catheter, can be coated from all sides by said process.

The coating is very homogenous as compared to other processes such as DC- or RF-sputtering. The interleaved PBII is contributing to this effect.

Some suitable process parameters are listed in the following:

### First set of parameters:

Frequency of the first and second series of pulses: 100 Hz / 50 Hz
Delay between first and second pulses: 3.9 ms
Pressure: 1 Pa
Target material: Cu
Voltage amplitude and duration for each series of pulses: 750 V, 100 µs / 7,0 kV, 2 ms

### Second set of parameters:

Frequency of the first and second series of pulses: 625 Hz / 0,5 Hz
Delay between first and second pulses: 400 µs
Pressure: 0,5 Pa
Target material: Cu
Voltage amplitude and duration for each series of pulses: 900 V, 20 µs / 3,5 kV, 10 µs

### Example 2: Determination of depth profile as shown in Fig. 3

Depending on the process parameters, the inventors have generated different depth profiles compared to pure HiPIMS. Basically, all distributions of the copper in the surface that were observed follow the same pattern: High concentration in the area near the surface, exponential decay to greater depths. The difference between the individual profiles is mainly the depth and the amount of implanted copper (height of the profile).

The measurements were carried out by X-ray photoelectron spectroscopy (XPS; Kratos Analytical Ltd., Axis Ultra). The X-ray radiation for the measurement was Al-Kα with an energy of 1486.6 eV. The first measurement corresponds to the composition of the surface. After the first measurement, the surface in the measuring chamber was slightly eroded by means of Ar-ion bombardment (a few nm) and the measurement was restarted. Iteratively, a depth profile is obtained. From the XPS spectrum only the range of about 925 eV to 970 eV was used for the determination of the intensity (area under the curve). This range can be assigned to copper and its oxides. The software CasaXPS can be used to create a surface fit which is subtracted from the entire measurement and then the actual curve is fitted. This results in the area under the peak and thus a value for the amount of copper in the surface.

### Example 3 Determination of crystallinity as shown in Fig. 5

Crystallinity was determined by X-ray diffractometry (XRD) of Cu from the same process on Si-Wafer by glazing incident diffractometry.

Measurements were performed using a 'Theta-2-Theta diffractometer HZG4' (Seifert FPM). In this device, a copper anode is used for the generation of the X-ray radiation. The Cu-Kα radiation for the measurement is selected via a graphite monochromator. The glazing incident diffractometry (0.5 °, 1 °) was measured in an angle range of 20-80 ° / 2Theta. The step width was 0.02 °, the integration time was 2 seconds.

### Example 4: Size of crystallites

The combination process produces larger crystallites. The crystal structure influences the release of the copper.

The inventors can therefore adjust the effect by tuning the crystallization between a shortlasting effect i.e., rapid and pronounced in quantity, and a long lasting effect i.e., slow and scarce. A difference of a few weeks can be asserted between the short- and the long-lasting effect in a blood environment (tested in simulated body fluid SBF; see Kokubo et al., (1990). Journal of Biomedical Materials Research. 24: 721-734).

In distilled water, the release of the copper is reduced by a factor of 10, while at the same time 10 times longer duration of the action. Thus, the effect also depends strongly on the ambient environment of the layer or the coated product.

The crystallite size is determined by means of the program Diffrac. Eva (Bruker).

### See Table:

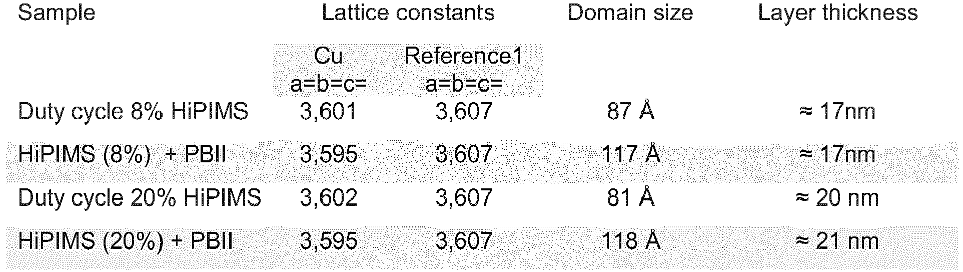

### Example 5: Anti-microbial effect as illustrated in Fig. 6

Acryolonitril-butadiene-styrene (ABS) plates treated with the method according to the invention (see set of parameters given above) were spread with 50µl of a suspension of Staphylococcus aureus (1,86E7 colony forming units per ml) in 0,85% aqueous buffered NaCl. The bacterial suspension remained 2h on the surface, after which the supernatant was collected and plated in dilutions for 24h at 37°C. The colony-forming units were counted. The result is given in Fig. 6. The treatment of the polymer with the method according to the invention leads to a six order of magnitude reduction of bacterial load.

### Example 6: Depth profile for copper ions

In Fig. 9 three depth profiles for silicon catheters treated with the method according to the invention are shown. Copper ions are incorporated into the catheter with the method according to the invention. Two sequences of pulses are used according to the invention, wherein the first sequence corresponds to a HiPIMS method and the second sequence corresponds to a PBII method. The three catheters were treated with the following parameters.
Pressure: 1 Pa
Target material: Cu
Parameters for the first sequence of pulses, i.e., the HiPIMS method:
   Voltage amplitude: 750 V;
   Pulse duration: 100 µs;
   Pulse frequency: 100 Hz.
Parameters for the second sequence, i.e., the PBII method:
   Voltage Amplitude: 7kV
   Pulse duration: 2 ms
   Pulse frequency: 50 Hz.

For the first catheter the method according to the invention was repeated for 5 min (plot with squares), for the second catheter the method according to the invention was executed for 10 min (circles) and for the third catheter the method according to the invention was executed for 15 min (triangles).

The concentration of the copper ions at the outer layer of the surface of the catheter is between 1% and 5% and then increases to a concentration of 15 % (5 min treatment) at a depth of 20 nm. For the second and third catheter the copper ion concentration increases to 32% and 36% respectively.

From these maximum values the concentration decreases exponentially for greater depths. If the catheter is treated for 5 min the concentration of copper ions vanishes at a depth of approximately 120 nm, wherein for the other catheter the concentration of copper ions vanishes at depths of 250 to 350 nm.

Fig. 9 shows how the concentration of copper ions at the surface and in the inner bulk material can be adjusted by changing the treatment duration of the catheter.

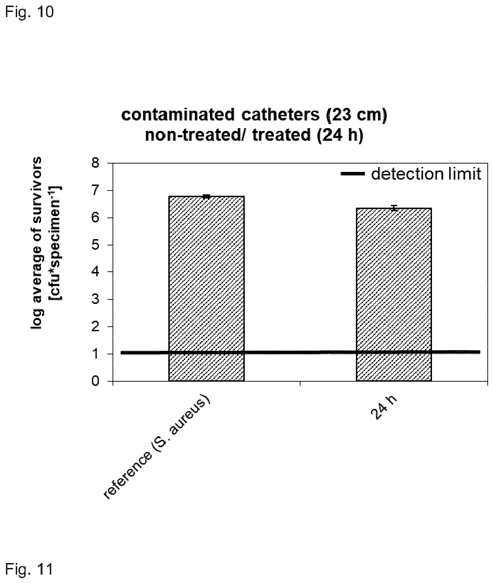

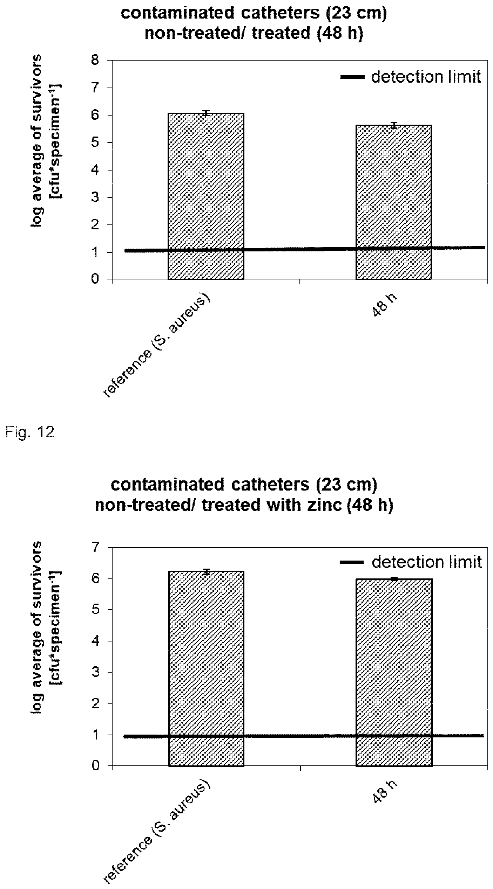

Fig. 10 and Fig. 11 show the antibacterial effect of a catheter that has been treated according to the method of the invention for 10 min with the parameters as disclosed in the previous example of Fig. 9. The logarithm of the estimated cfus (colony forming units) of the bacterium staphylococcus aureus on a non-treated catheter (left bar) and the treated catheter (right bar) are shown after 24 h (Fig. 10) and after 48 h (Fig. 11). It can be seen that the treated catheter has a 40% to 190% times lower number of cfus (right bar). This is due to the anti-bacterial effect of the copper that is imbedded in the surface region of the treated catheter.

In Fig. 12 the anti-bacterial effect on staphylococcus aureus of a catheter that has been treated with zinc instead of copper is shown after 48h. Also here, the effect can be detected, however to lower degree. The treated catheter (right bar) shows 25% less cfus of staphylococcus aureus than the non-treated catheter (left bar).

### References:

[1] Lundin, D. and Sarakinos, K. (2012) 'An introduction to thin film processing using high-power impulse magnetron sputtering', Journal of Materials Research, 27(5), pp. 780-792. doi: 10.1557/jmr.2012.8.

## Claims

1. A medical device comprising a biocompatible material having an exposed surface, wherein said biocompatible material comprises a doping of an antibacterial metal compound,
**characterized in that**
a concentration of said doping compound in said biocompatible material ranges from 1% to 30% (w/w) within an outer layer adjacent to said exposed surface, and is essentially zero within a bulk layer of said biocompatible material.

2. The medical device according to claim 1, wherein said concentration of the doping compound
a. has a maximum value at said exposed surface and decreases linearly or exponentially to said doping compound being undetectable at a depth of 10 nm to 1000 nm, particularly at a depth of 100 nm to 500 nm, more particularly at a depth of 100 nm to 300 nm; or
b. has a maximum value at a depth of 10 to 500 nm; and decreases linearly or exponentially to said doping compound being undetectable at a depth of 10 nm to 1500 nm, particularly at a depth of 100 nm to 300 nm; or
c. is undetectable on the surface, has a maximum value at a depth of 10 nm to 500 nm, particularly in a depth of 100 nm to 300 nm; and decreases linearly or exponentially at a depth greater than the depth of maximum concentration value until said doping compound is undetectable at a depth of 800nm, 1000 nm or 1500 nm.

3. The medical device according to any one of the preceding claims, wherein said antibacterial metal compound is selected from copper, copper oxide, silver, zinc, zinc oxide, cobalt, cobalt oxide, aluminium, aluminium oxide, titanium, titanium oxide, and mixtures thereof.

4. The medical device according to any one of the preceding claims, wherein said exposed surface comprises or is essentially made of
a. a material selected from silicon, particularly medical silicon, polyurethane (particularly aliphatic polyurethane, more particularly thermoplastic aliphatic polyurethane), polyvinylchloride, polypropylene, polystyrene, latex, teflon or a mixture of these materials,
b. a shape memory alloy, particularly NiTiNol or NiTiCo or
c. a load bearing material, particularly selected from titanium or a titanium alloys used as load bearing implants as specified in ASTM Grade 1-35, stainless steel, and a resorbable material, particularly a magnesium alloy or hydroxy apatite.

5. The medical device according to any one of the preceding claims, wherein a coating, particularly a coating having a thickness ranging from 1 nm to 400 nm, more particularly ranging from 10 nm to 100 nm, of said antibacterial metal compound is comprised on said exposed surface.

6. The medical device according to claim 5, wherein the antibacterial compound of said coating is the same as the antibacterial compound comprised in said outer layer.

7. The medical device according to any one of the preceding claims, wherein the medical device is a urinary tract catheter.

8. The medical device according to claim 7, wherein said exposed surface is an outer surface opposite to a luminal inner surface.

9. The medical device according to any one of the previous claims, wherein said medical device comprises a lumen enclosed by a surface, and said exposed surface is an outer surface opposite to a luminal inner surface.

10. A process for treating a biocompatible material having an outer layer comprising a doping of an antibacterial metal compound, said process comprising the steps of
a. mounting a biocompatible material on an electrode in an enclosed space of a coating system comprising a target material and a magnetron source for magnetron sputtering;
b. reducing the atmospheric pressure in said enclosed space to below 1 Pa particularly to below 0.1 Pa, 0.01 Pa or even 0.001 Pa;
c. applying a first series of negative voltage pulses to the magnetron source and/or the target so as to create a plasma within said enclosed space, whereby said target material is ionized and released into said chamber;
d. applying a second series of negative voltage pulses to said electrode, particularly to the biocompatible material, wherein the negative voltage pulses of the second series are time-delayed with respect to the voltage pulses of the first series by at least the duration a voltage pulse of the first series, wherein the ionized target material is accelerated towards said electrode and implanted in the biocompatible material.

11. The process according to claim 10, wherein said target material is copper, copper oxide, silver, zinc, zinc oxide, cobalt, cobalt oxide, aluminium, aluminium oxide or a mixture thereof.

12. The process according to any one of claims 10 or 11, wherein the creation of ionized target material is facilitated by a pulsed magnetron sputtering method.
